(19) Europäisches Patentamt
European Patent Office
Office européen des brevets

(11) Publication number: **0 479 553 B1**

(12) **EUROPEAN PATENT SPECIFICATION**

(45) Date of publication of patent specification: **12.04.95**

(51) Int. Cl.⁶: **C04B 38/00**, B01D 39/20, B01D 71/02, C12N 11/14

(21) Application number: **91308980.1**

(22) Date of filing: **01.10.91**

(54) **Production of porous ceramics.**

(30) Priority: **01.10.90 JP 262778/90**

(43) Date of publication of application:
**08.04.92 Bulletin 92/15**

(45) Publication of the grant of the patent:
**12.04.95 Bulletin 95/15**

(84) Designated Contracting States:
**DE GB**

(56) References cited:
**FR-A- 2 550 782**
**FR-A- 2 587 231**
**GB-A- 285 470**
**GB-A- 542 559**
**US-A- 3 233 011**

**CHEMICAL ABSTRACTS, vol. 107, no. 6, August 1987, Columbus, Ohio, US; abstract no. 45110E, O. MURAYAMA ET AL.: 'Porous ceramics' page 335; & JP-A-62 087 472**

(73) Proprietor: **SAGA PREFECTURE**
**1-1-59 Jounai**
**Saga-shi,**
**Saga 840 (JP)**

(72) Inventor: **Ichinose, Hiromichi**
**1516, Oaza-Tanoue,**
**Ariake-cho**
**Kishima-gun,**
**Saga 849-12 (JP)**
Inventor: **Kawahara, Akihiko**
**26-12, Tanoura-cho,**
**Sasebo-shi**
**Nagasaki 859-32 (JP)**
Inventor: **Katsuki, Hiroaki**
**1-1607-22, Setogoe,**
**Sasebo-shi**
**Nagasaki 857-01 (JP)**
Inventor: **Furuta, Sachiko**
**1058, Chubuhei,**
**Arita-cho**
**Nishi Matsuura-gun,**
**Sega 844 (JP)**
Inventor: **Nakao, Hiroshi**
**23498, Oaza-Miyano,**
**Yamauchi-cho**
**Kishima-gun,**
**Saga 849-23 (JP)**

(74) Representative: **Moore, Anthony John et al**
**Gee & Co.**
**Chancery House**
**Chancery Lane**
**London WC2A 1QU (GB)**

## Description

The present invention relates to a method of producing porous ceramics, particularly porous ceramics which have high porosity and a narrow pore size distribution. The method of the present invention is particularly useful for producing various types of catalyst carriers, ceramic membranes, filter plates, bioreactors and sensors, and other elements used at high temperatures.

So far, porous ceramics have been produced by regulating the pore size of a batch of raw particulate material, compacting or otherwise forming it, and calcining it to regulate the interstices between pores in the green ceramic body, thereby producing a porous ceramic body; or alternatively firing a batch of raw material containing an organic material such as synthetic fiber or cellulose to burn out that organic material, thereby forming pores and obtaining a porous ceramic body. In order to obtain ceramic porous bodies by these ceramic-forming techniques, the starting batch is formed by ceramic forming procedures similar to those for obtaining tightly compacted ceramic bodies, such as pressing, extrusion or casting, followed by firing.

However, a problem with using ceramic forming procedures similar to currently available ones for obtaining tightly compacted, sintered ceramic bodies is that the forming density is so increased that the proportion of discrete particles is considerably reduced. This results in another problem that when sintering is caused to proceed further by post-firing to increase the strength of the porous ceramic compacts, their porosity drops to about 30 to 50% due to shrinkage on firing. In general, a ceramic raw material is wet formed or compacted at a concentration of 60% or more with respect to the slurry batch used. The compact obtained in this case, however, is so increased in density that the proportion of discrete particles is significantly reduced. In consequence, the porosity-on-drying of the compact drops sharply upon firing at high temperature with the possible occurrence of closed pores; considerable difficulty has been encountered in obtaining porous ceramics having high porosity, uniform pore size and excellent mechanical strength. Thus, porous ceramic bodies having the good properties that their precursors or compacts have as such, e.g. good pore structure and pore distribution, have less than satisfactory mechanical strength; it is only porous ceramics having low mechanical strength that can now be obtained.

In order to achieve high porosity by the technique for manufacturing porous ceramics from a ceramic batch containing organic material such as synthetic fiber or cellulose, it is required to use them in large quantities, but this poses some problems in that it is difficult to disperse the organic materials uniformly throughout the ceramic raw material; and the resulting size distribution of pores is broad. The organic materials also make it impossible to increase heating rates, and it takes much time to burn them out.

Other techniques for obtaining porous ceramic bodies are also available, one of which comprises preparing a metal alkoxide, a typical ceramic raw material which serves as an aggregate, causing it to gelate by hydrolysis, and finally drying and calcining the gel product; and another of which comprises coating or spraying a ceramic slurry of a metal alkoxide sol onto a ceramic carrier, followed by calcination.

However, problems with using the metal alkoxide as the ceramic raw material are that they are costly and awkward to produce, and that the porosity of the resulting porous compact drops extremely upon being fired at a temperature of 1000°C or higher.

In order to obtain porous ceramic films by coating or spraying an ordinary ceramic raw material slurry thinly onto a support, it is required to decrease its viscosity. However, a problem with reducing the solids content of the ceramic raw material slurry to decrease its viscosity is that the ceramic raw material aggregates, making the thickness of the film uneven and giving rise to pin holes.

According to the present invention a porous ceramic having high porosity and a narrow pore size distribution is obtained by bringing a dilute slurry, obtained by mixing a ceramic raw material, present in said slurry at a concentration of from 1 to 60% by weight, with a solution of an organic high-molecular compound, that is capable of gelating through reaction with an acid or a di- or more-valent cation or by being heated or cooled, into contact with a liquid or gel in which an acid or a di- or more-valent cation is present, or heating or cooling said dilute slurry to prepare a gel compact, and firing the thus obtained gel compact. A hybrid porous ceramic may be obtained from the ceramic raw material in the slurry and the di- or more-valent cation compound used to instigate gelation.

Some solutions of naturally occurring or synthetic high-molecular substances are known to gelate through chemical reaction or on heating or cooling. As noted earlier, in the method of the present invention a particulate ceramic raw material is added to a solution of a naturally occurring or synthetic compound capable of gelating as a result of a chemical reaction, or by being heated or cooled to prepare a slurry, and pouring said slurry into, or otherwise bringing it into contact with, a liquid or gel in which an acid or a di- or more-valent cation is present, or heating or cooling said slurry to achieve gelation.

3

EP 0 479 553 B1

In the method of this invention, the ceramic raw particles are each surrounded on gelation by a large amount of formed solvent, because the large molecules are cross-linked together. In consequence, any rearrangement of the slurry is very unlikely to occur even after gel drying; thus it is possible to obtain a green ceramic body having an increased proportion of discrete particles and a very greatly increased porosity.

When the gelated green ceramic body is dried and then fired by heating to a temperature higher than the sintering temperature of the ceramic raw material used, the overall sintering area is so limited due to the increased proportion of discrete particles that the open pores are substantially left intact, thus making it possible to obtain porous ceramics in which the particulate material is tightly bonded together around pores, and which have high porosity and excellent mechanical strength. The green ceramic body must be fired by heating to a temperature higher than that at which the sintering of the ceramic raw material begins to take place. Although it may be possible to vary the pore characteristics of the resulting ceramic product without causing any significant change in its porosity by altering the firing temperature and time, it is preferred that the firing temperature lie in the range between the temperature at which the sintering of the ceramic raw material begins to take place and up to the temperature at which a perfectly sintered, densified ceramic product is obtained.

Thus, the porous ceramics of this invention suffer no change in their pore characteristics even upon use at high temperatures; they can be used as catalyst carriers for high-temperature purposes and as bioreactors needed to have wear resistance and compression strength. According to this invention, it is also possible to obtain various filters for fluids or gases - which have no or little pinholes, are very thin and excel in fluid premeability, typically air filters or filters for fermented yeasts, by coating a gelating slurry containing ceramic particles onto a suitable ceramic support to form a film, followed by the gelation of the film.

For instance, the organic high-molecular compounds which are used for obtaining the gel-forming slurry according to this invention and can gel in the presence of an acid or a di- or more-valent cation include alginates such as sodium and ammonium alginates, $x$-carrageenan, $\iota$-carrageenan and pectin, each used in the form of an aqueous solution. It is preferred that the aqueous solution of the organic high-molecular compound be used at a concentration of 0.1 to 5 % by weight. This is because concetrations below 0.1 % by weight would give rise to a lowering of gel strength while concentrations higher than 5 % by weight would make the viscosity of the slurry too high to use it.

In order to give form to the slurry by gelation, a given quantity of the slurry may be poured in a liquid containing an acid or a di- or more-valent cation that is a gelling agent, or may otherwise be brought into contact with that liquid. In this way, any desired, e.g. particulate, thread-like, sheet-like or bulky, form of green ceramic body may be obtained. In order to obtain a film-form porous ceramic, the slurry may be coated onto a suitable porous ceramic support and then brought into contact with a liquid containing an acid or a di- or more-valent cation that is a gelling agent, as already mentioned.

When acids are used as the gelling agent, most acids, whether inorganic or organic, may be employed, but preference is given to using acids having a pH value of 3 or less. At pH higher than 3 no instantaneous gelation would take place. Acids that volatilize off during firing and so are unlikely to contaminate porous ceramics with impurities may also be used.

When liquids containing di- or more-valent cations are used as the gelling agent, preference is given to using aqueous solutions of chlorides, sulfates and nitrates of calcium, aluminium, zirconium, barium, chromium, etc. Preferably, such aqueous solutions have a salt concentration of at least $1 \times 10^{-3}$ mol/l, below which difficulty would be encountered in instantaneous gelation.

In order to enhance the dispersibility of the ceramic particulate material, the slurry may additionally and preferably contain generally available dispersants, typically ammonium polycarbonate, ammonium polyacrylate, polyacrylic ester and condensed ammonium naphthalene-sulfonate.

For solutions of high-molecular substances that may be used for the method for producing porous ceramics according to this invention and gelate by heating, preference is given to using methylcellulose, carboxymethylcellulose, etc. at a concentration of 0.1 to 5 % by weight. Giving form to the slurry - obtained by dispersing the ceramic raw material in a solution of the high-molecular substance capable of gelating by heating - by gelation may be achieved, by way of example, by heating the slurry filled in a mold or pouring or spraying the slurry into or onto hot water of 50-100°C. What procedures are chosen for gelation suitability depends upon the required shape and size.

Substances usable for the method for producing porous ceramics according to this invention and capable of gelating by cooling, for instance, include polysaccharides such as carrageenan, agar and xanthane gum, gelatin and polyvinyl alcohol.

The gelling procedures by heating or cooling are best suited for obtaining thick green ceramic bodies.

4

The present invention will now be illustrated by the following non-limiting Examples.

Example 1

One hundred (100) parts by weight of high-purity alumina with an average particle size of 0.2 $\mu$m (TMD made by Taimei Kagaku Kogyo K. K.) and 120 parts by weight of a 1 wt.% aqueous solution of ammonium alginate were mixed together with 1.8 parts by weight of an ammonium polycarboxylate dispersant (Aron A-6114 made by Toa Gosei Kagaku Kogyo K.K.) in a container for 3 hours in the presence of partially stabilized zirconia balls, and the mixture was then aged to 24 hours to prepare a slurry having an alumina content of about 45 %. Packed in an injector, this slurry was passed through its 2-mm inside diameter nozzle into an aqueous solution of aluminium sulfate with a 0.05 mol/l concentration. After allowing to stand for 20 minutes, it was washed with distilled water and then dried at 60°C for 3 hours.

As measured after drying, the green ceramic body was found to have a mean pore size of 0.18 $\mu$m, a porosity of 71 % and a compression strength of 50 kg/cm$^2$. Then, the green body was fired at temperatures of from 1100°C to 1350°C for 1 hour to obtain porous ceramics. The relations between the firing temperature and the apparent porosity and compression strength of the obtained porous ceramics are set out in Table 1. Note that they were all found to have a constant mean pore size of 0.15 $\mu$m irrespective of at what temperatures they had been fired.

Comparative Example 1

Distilled water was added to 100 parts by weight of high-purity alumina with a mean particle size of 0.2 $\mu$m (TMD made by Taimei Kagaku Kogyo K. K.) and 1.8 parts by weight of an ammonium polycarboxylate dispersant (Aron A-6114 made by Toa Gosei Kagaku Kogyo K.K.) to prepare a slurry having an alumina content of ca. 70 % by weight, which was in turn mixed together for 3 hours as in Example 1, followed by 24-hour aging. The thus obtained slurry was cast in a gypsum mold to obtain 50 mm x 50 mm x 50 mm green ceramic bodies. After drying at ambient temperature for 12 hours and then at 60°C for a further 3 hours, they were fired at temperatures of from 1050°C to 1350°C for 1 hour. The relation between the firing temperature and the apparent porosity is set out in Table 1. The porous ceramics, fired at temperatures of 1050°C or higher, were all found to have a compression strength of 250 kg/cm$^2$.

Comparative Example 2

One hundred (100) parts by weight of high-purity alumina with an average particle size of 0.2 $\mu$m (TMD made by Taimei Kagaku Kogyo K. K.) and 43 % by weight of a 0.5 wt.% aqueous solution of ammonium alginate were mixed together with 1.8 parts by weight of an ammonium polycarboxylate dispersant (Aron A-6114 made by Toa Gosei Kagaku Kogyo K.K.) for 3 hours as in Example 1, and the mixture was then aged for 24 hours to prepare a slurry having an alumina content of ca. 70 %.

Packed in an injector, this slurry was passed through its 2-mm inside diameter nozzle into an aqueous solution of aluminium sulfate with a 0.05 mol/l concentration. After allowing to stand for 20 minutes, it was washed with distilled water and then dried at 60°C for 3 hours.

Following drying, the dried body was fired at temperatures between 1050°C and 1350°C.

## Table 1

| Firing Temp. (°C) | Example 1 | | Comp. Ex. 1 | | Comp. Ex. 2 | |
|---|---|---|---|---|---|---|
| | A.P. (%) | C.S. (kg/cm$^2$) | A.P (%) | C.S. (kg/cm$^2$) | A.P. (%) | C.S. (kg/cm$^2$) |
| 1050 | -- | | 42 | 250 | 44 | 200 |
| 1100 | 63 | | 40 | | -- | |
| 1150 | -- | | 37 | | 39 | |
| 1200 | 59 | | -- | | -- | |
| 1250 | -- | | 15 | | 18 | |
| 1350 | 44 | 1500 | 0 | | 0 | |

Note that A.P. and C.P. stand for apparent porosity and compression strength, respectively.

The above data indicate that the porous ceramics obtained according to the method of this invention have a sufficiently high porosity even at the temperature at which the cast ceramic bodies are perfectly sintered, and shows an increased mechanical strength as well.

### Example 2

Forty (40) % by weight of high-purity $\gamma$-alumina with a specific surface area of 140 m$^2$/g (AKP-G made by Sumitomo Chemical Co., Ltd.) and 360 parts by weight of a 2 wt.% aqueous solution of ammonium alginate were mixed together with 4 % by weight of an ammonium polycarboxylate dispersant (Aron A-6114 made by Toa Gosei Kagaku Kogyo K.K.) and then aged as mentioned in Example 1. Filled in an injector, the slurry having an alumina content of ca. 10 % was passed through its 2-mm diameter nozzle into 1N hydrochloric acid, allowed to stand for 20 minutes, washed with distilled water and dried at 60°C for 3 hours.

Then, the dried bodies were fired at temperatures between 1100°C and 1500°C to obtain porous ceramics.

The sintering temperature, apparent porosity and pore size of each of the obtained porous ceramics are set out in Table 2.

### Example 3

Filled in an injector, the same slurry as prepared in Example 2 was passed through its 2-mm nozzle into an 0.05 N aqueous solution of zirconium oxychloride, permitted to stand for 20 minutes, washed with distilled water and dried at 60°C for 3 hours.

The obtained dried bodies were fired at temperatures between 1350°C and 1500°C for 1 hour to obtain porous ceramics.

The firing temperature, apparent porosity and pore size of each of the obtained porous ceramics are set out in Table 2.

Accompanying the present specification is an electron micrograph of 20,000 magnifications, showing a section of the porous ceramic specimen obtained at 1350°C. As can be best shown, the alumina matrix is in an entangled state, as is the case with porous glass, retains a void content higher than would be possible

6

using conventional forming technique and consists of a three-dimensional network structure. Moreover, not only does this ceramic specimen no longer retain the pore characteristics of the green ceramic body but it is also increased in strength due to even more enhanced interparticle sintering. In addition, within the porous ceramic structure there are uniformly and finely dispersed zirconia particles.

## Table 2

| Firing Temp. | Example 2 | | Example 3 | |
|---|---|---|---|---|
| | A.P. | M.P.S. | A.P. | M.P.S. |
| (°C) | (%) | (μm) | (%) | (μm) |
| 1100 | 78 | 0.03 | —— | |
| 1200 | 72 | 0.16 | —— | |
| 1350 | 68 | 0.40 | 64 | 0.18 |
| 1500 | 63 | 0.57 | 58 | 0.38 |

Note that A.P. and M.P.S stands for apparent porosity and mean pore size, respectively.

Example 4

The slurry prepared in Ex. 1 was coated onto the inner surface of an 8-mm inside diameter pipe formed of alumina with a pore size of ca. 2 μm, which was in turn immersed in 1N hydrochloric acid. After allowed to stand for 5 minutes, the pipe was washed with distilled water and then dried at 60°C for 3 hours. The pipe was subsequently fired at 1200°C for 1 hour to obtain a ceramic filter with a porous ceramic film thereon. No pinholes were observed at all on the smooth surface of the ceramic film, which was found to have a pore size of 0.15 μm and a thickness of 3 μm, both good enough.

Example 5

The slurry prepared in Ex. 2 was coated onto the inner surface of an 8-mm inside diameter pipe formed of alumina with a pore size of ca. 2 μm, which was in turn immersed in 1N hydrochloric acid. After allowing to stand for 1 minute, the pipe was washed with distilled water and then dried. The pipe was subsequently fired at a temperature varying between 1100°C and 1500°C for 1 hour to obtain a ceramic filter with a porous ceramic film thereon. No pinholes were observed at all on the smooth surface of each ceramic film, which was found to have a satisfactory thickness of ca. 3 μm. The size pores were substantially the same as set out in Table 2.

Example 6

A powdery mixture of 72 % of intermediate alumina powders with high purity and a specific surface area of about 140 m²/g (AKP-G made by Sumitomo Chemical Co., Ltd.) with 28 % of silica powders with a medium particle size of 0.5 μm was mixed with a 1 % aqueous solution of ammonium alginate, and the mixture was then aged into a slurry containing about 30 % of the ceramic raw material.

The above slurry was coated onto the inner surface of an 8-mm inside diameter pipe formed of alumina with a pore size of 2 μm. Then, the alumina pipe was immersed in 1N hydrochloric acid, permitted to stand for 1 minute, washed with distilled water and dried. After that, it was fired at 1600°C for 2 hours to obtain a filter with a porous mullite film thereon. No pinholes were observed at all on the smooth surface of the film, which was found to have a pore size of 0.80 μm and a thickness of 10 μm, each good enough. Because of

7

firing at high temperature, the film was converted into a single phase consisting substantially of mullite, suggesting that it would be useful as a filter excellent in heat resistance but also for other purposes.

Example 7

One hundred (100) parts by weight of high-purity $\alpha$-alumina with a mean particle size of 0.2 $\mu$m (TMD made by Taimei Kagaku Kogyo K.K.), 120 parts by weight of a 3 % aqueous solution of $x$-carrageenan, heated to 95°C, and 1.8 parts by weight of a dispersant (Aron A-6114 made by Toa Gosei Kagaku Kogyo K.K.) were heated and stirred together for 3 hours to prepare a slurry. This slurry was charged in a 10 mm x 10 mm x 10 mm frame, allowed therein to stand for 3 hours, cooled off to an ambient temperature of 30°C or lower, removed from the frame and dried in vacuo. After that, the green ceramic body was fired at 1350°C for 1 hour to obtain a porous ceramic product, which was found to have a porosity of 45 % and a mean pore size of 0.15 $\mu$m.

Example 8

One hundred (100) parts by weight of high-purity $\alpha$-alumina with a mean particle size of 0.2 $\mu$m (TMD made by Taimei Kagaku Kogyo K.K.), 120 parts by weight of a 2 % aqueous solution of methylcellulose and 1.8 parts by weight of a dispersant (Aron A-6114 made by Toa Gosei Kagaku Kogyo K.K.) were heated and stirred together for 3 hours to prepare a slurry. This slurry was charged in a 10 mm x 10 mm x 10 mm frame wherein it was then heated at 90°C for 1 hour for gelation. After removal from the frame, the gel body was dried at 70°C for 3 hours and then fired at 1350°C for 1 hour to obtain a porous ceramic product, which was found to have a porosity of 43 % and a mean pore size of 0.15 $\mu$m.

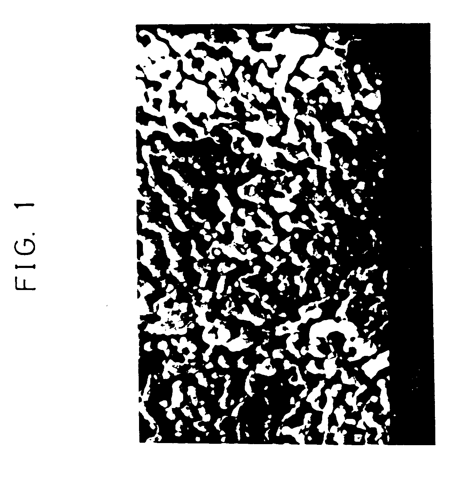

## Claims

1. A method for producing a porous ceramic product by forming and firing a ceramic raw material, characterized in that the method comprises the steps of mixing said ceramic raw material with a solution of an organic high-molecular weight compound capable of being gelated by reaction with a solution of an acid or a di- or more-valent cation, or by being heated or cooled, to prepare a slurry containing said ceramic raw material at a concentration of from 1 to 60% by weight, contacting such slurry with a solution containing an acid or di- or more-valent cation, or heating or cooling said slurry, thereby obtaining a green ceramic body, and drying and firing said green ceramic body.

2. A method as claimed in Claim 1, wherein said di- or more-valent cation is incorporated into said porous ceramic product.

3. A method as claimed in Claim 1 or 2, wherein said porous ceramic product is a filter for liquids or gases.

4. A method as claimed in Claim 3, wherein said slurry contains a solution of an organic high molecular weight compound capable of being gelated by reaction with an acid or a di- or more-valent cation, and the slurry is coated onto a porous ceramic support before being contacted with said solution containing an acid or di- or more-valent cation.

5. A method as claimed in any preceding claim, characterized in that said porous ceramic product is a carrier for bioreactors.

## Patentansprüche

1. Verfahren zur Herstellung eines porösen keramischen Produkts durch Formung und Brennen eines keramischen Rohmaterials, dadurch gekennzeichnet, daß das Verfahren die Schritte umfaßt, daß das besagte keramische Rohmaterial mit einer Lösung einer organischen Verbindung hohen Molekulargewichts gemischt wird, welche in der Lage ist, durch Reaktion mit einer Lösung einer Säure oder eines zwei- oder mehrwertigen Kations oder durch Erhitzen oder Abkühlen zu glieren, um einen Brei herzustellen, der das genannte keramische Rohmaterial in einer Konzentration zwischen 1 und 60 Gew.-% enthält, daß dieser Brei mit einer Lösung versetzt wird, die eine Säure oder ein zwei- oder mehrwertiges Kation enthält oder daß der Brei erhitzt oder gekühlt wird, um dadurch einen grünen keramischen Scherben zu erhalten, und daß dieser Scherben getrocknet und gebrannt wird.

2. Verfahren nach Anspruch 1, dadurch gekennzeichnet, daß das zwei- oder mehrwertige Kation in das poröse keramische Produkt eingebettet ist.

3. Verfahren nach Anspruch 1 oder 2, dadurch gekennzeichnet, daß das poröse keramische Produkt ein Filter für Flüssigkeiten oder Gase ist.

4. Verfahren nach Anspruch 3, wobei der genannte Brei eine Lösung einer organischen Verbindung hohen Molekulargewichts enthält, die in der Lage ist zu gelieren durch Reaktion mit einer Säure oder einem zwei- oder mehrwertigen Kation, und der Brei auf einem porösen keramischen Träger geschichtet wird, bevor er mit der genannten Lösung einer Säure oder eines zwei- oder mehrwertigen Kations in Kontakt tritt.

5. Verfahren nach einem der vorhergehenden Ansprüche, dadurch gekennzeichnet, daß das genannte poröse keramische Produkt ein Träger für Bioreaktoren ist.

**Revendications**

1. Procédé de production d'un produit céramique poreux par formage et chauffe d'un matériau céramique brut, caractérisé en ce que le procédé comprend les étapes de mélange dudit matériau céramique brut avec une solution d'un composé organique à poids moléculaire élevé capable d'être gélifié par réaction avec une solution d'un acide ou d'un cation bivalent ou multivalent ou en étant chauffé ou refroidi, pour préparer un coulis contenant ledit matériau céramique brut à une concentration de 1 à 60% en poids, mise en contact de ce coulis avec une solution contenant un acide ou un cation bivalent ou multivalent, ou chauffage ou refroidissement dudit coulis, pour obtenir ainsi un corps céramique vert, et séchage et chauffe dudit corps céramique vert.

2. Procédé selon Revendication 1, dans lequel ledit cation bivalent ou multivalent est incorporé dans ledit produit céramique poreux.

3. Procédé selon Revendication 1 ou 2 dans lequel ledit produit céramique poreux est un filtre pour liquides ou gaz.

4. Procédé selon Revendication 3 dans lequel ledit coulis contient une solution d'un composé à poids moléculaire élevé capable d'être gélifié par réaction avec un acide ou un cation bivalent ou multivalent, et dans lequel le coulis est appliqué en couche sur un support céramique poreux avant d'être mis en contact avec ladite solution contenant un acide ou un cation bivalent ou multivalent.

5. Procédé selon l'une quelconque des revendications précédentes, caractérisé en ce que ledit produit céramique poreux est un support pour bioréacteurs.

FIG. 1